# EUROPÄISCHE PATENTSCHRIFT

(11) **EP 2 581 082 B1**
(45) Veröffentlichungstag und Bekanntmachung des Hinweises auf die Patenterteilung: **30.12.2015**
(21) Anmeldenummer: 11184502.0
(22) Anmeldetag: 10.10.2011
(51) Int. Cl.: A61K 47/02, A61K 47/10, A61K 9/08, A61K 31/4166, A61K 9/00, A61K 45/06

(54) **Pharmazeutische Zusammensetzung zur Behandlung von Status asthmaticus**
Pharmaceutical composition for treating status asthmaticus
Composition pharmaceutique pour le traitement d'un état de mal asthmatique

(43) Veröffentlichungstag der Anmeldung: 17.04.2013
(73) Patentinhaber: Carinopharm GmbH, 31008 Elze (DE)
(72) Erfinder: Dr. Beute, Jan, 1355 HK Almere (NL)
(74) Vertreter: Becker Kurig Straus

(56) Entgegenhaltungen:
- US-A- 4 405 635
- US-A1- 2006 292 213
- DESMOND J. YOUNG, GARY A. SALZMAN: "Status Asthmaticus in Adult Patients", HOSPITAL PHYSICIAN, Bd. 2006, 1. November 2006 (2006-11-01), Seiten 13-24, XP002669476, Wayne
- WIEBALCK ET AL: ENOXIMON IM KLINISCHEN ALLTAG /// ENOXIMONE IN THE CLINICAL DAILY ROUTINE, CARINOPHARM , 24. Oktober 2011 (2011-10-24), Seiten 1-12, XP009155500, Gefunden im Internet: URL:http://www.carinopharm.de/start/downlo ad/2011_carinopharm_Infobroschu ere.pdf [gefunden am 2012-02-07]

## Beschreibung

### Gebiet der Erfindung

Die vorliegende Erfindung betrifft eine pharmazeutische Zusammensetzung in Form einer intravenös verabreichbaren, Enoximon enthaltenden Lösung zur Verwendung in der Behandlung von Status asthmaticus.

### Stand der Technik

Asthma bronchiale, oder vereinfachend auch Asthma genannt, ist eine chronische, entzündliche Erkrankung der Atemwege mit dauerhaft bestehender Überempfindlichkeit. Bei entsprechend veranlagten Personen führt die Entzündung zu anfallsweiser Luftnot infolge einer Verengung der Atemwege, einer sogenannten Bronchokonstriktion. Die Atemwegsverengung wird durch vermehrte Sekretion von Schleim, Verkrampfung der Bronchialmuskulatur und Bildung von Ödemen der Bronchialschleimhaut verursacht. Sie ist durch eine entsprechende Behandlung rückbildungsfähig. Zur Behandlung von Asthma werden oftmals Medikamente, wie beispielsweise Beta-2-mimetische Wirkstoffe, und/oder anticholinergene Wirkstoffe, mittels eines Zerstäubers verabreicht. Bei einem schweren Asthmaanfall kann eine Nebulisierung von Wirkstoff aber fehlschlagen, da die Wirkstoffe die Aveoli nicht mehr erreichen können.

Bei Status asthmaticus, auch bekannt als akutes schweres Asthma bronchiale, handelt es sich um eine schwere, anhaltende Symptomatik eines Asthmaanfalls über einen Zeitraum von bis zu 24 Stunden, hervorgerufen durch einen starken Spasmus der Bronchien. In besonders schweren Fällen kann es auch zu einem Atemstillstand kommen. Die bei Asthma sonst üblichen therapeutischen Maßnahmen sind dabei nur bedingt anwendbar.

Symptome des akuten schweren Asthmas bronchiale sind Kurzatmigkeit (Dyspnoe) mit hechelnder, beschleunigter Atmung und eine Abschwächung des Atemgeräusches in Folge der Verengung der Bronchien. Durch die Unterversorgung des Körpers mit Sauerstoff (Hypoxie) kann es zu Blässe, Blaufärbung der Lippen, der Finger und Zehen und zu Bewusstseinsstörungen kommen. Der Körper versucht, die Hypoxie zu kompensieren, in dem er die Herzfrequenz erhöht. Dies kann zu Herzarrhythmien führen.

Wichtigstes diagnostisches Kriterium bei einem Asthmaanfall ist, neben den vorstehend genannten Symptomen, die arterielle Blutgasanalyse, die Aufschluss über die Menge des im Blut enthaltenen Sauerstoffs sowie des Kohlendioxids und damit den Schweregrad des Asthmas gibt.

Behandelt wird Status asthmaticus normalerweise beispielsweise durch Sauerstoffgabe, einer Verabreichung von Beta-2-Sympathomimetika zur Weitung der Bronchien, Aminophyllin und/oder Theophyllin zur Behebung des Bronchospasmus, Corticosteroiden zur Hemmung der Entzündungsreaktion sowie Parasympatholytika, um die Kontraktion der glatten Muskulatur und die Schleimproduktion zu hemmen. In besonders schweren Fällen kann eine nichtinvasive Beatmung oder eine Beatmung mit endotrachealer Intubation und folgender maschineller Beatmung nötig werden. Eine künstliche Beatmung kann aber zu einer Vielzahl von Komplikationen führen, wie beispielsweise Infektionen und einer mechanische Schädigung des Lungengewebes.

Falls eine Intubation nicht verhindert werden kann, dauert es normalerweise drei bis fünf Tage bevor die Bronchokonstriktion wieder aufgehoben werden kann. Die respiratorische Behandlung von behandlungsresistentem Asthma kann so weit gehen, dass Anästhetika, wie beispielsweise Sevofluran, gegeben werden und mittels extrakorporaler Membranoxygenierung (ECMO) eine extrakorporale CO₂-Extraktion und Sauerstoffgabe durchgeführt werden müssen. Dies erfordert aber spezielle Einrichtungen und Notfallbehandlungszentren, da eine ECMO-Behandlung nur in Intensivstationen mit entsprechender Erfahrung durchgeführt werden sollte. Die Behandlung mit Anästhetika ist in der Praxis oftmals nur in Operationsräumen möglich, die aber nicht für die Behandlung von Intensivpatienten über einen längeren Zeitraum gedacht sind.

In den meisten Fällen werden jedoch die Patienten mit Status asthmaticus in normalen Krankenhäusern aufgenommen und zumindest die Initialbehandlung muss dort stattfinden. Eine vergleichbare Situation ergibt sich bei der Erstversorgung von Asthmapatienten durch einen Notarzt, insbesondere von Patienten mit Atemstillstand, vor Ort. Auch hier ist eine einfache Behandlungsmethode als Sofortmaßnahme wünschenswert.

Enoximon (INN, Handelsname Perfan^{®}) (1,3-Dihydro-4-methyl-5-[4-(methylthio)benzoyl]-2H-imidazol-2-on) ist ein bekannter Phosphordiesterase-III Inhibitor und wird bei der Behandlung von Herzversagen angewendet. Enoximon wird beispielsweise detailliert in der US 4,405,635 beschrieben. Die EP 0 326 103 B1 beschreibt weiterhin flüssige pharmazeutische Zusammensetzungen zur oralen Verabreichung, welche eine wirksame Menge von Enoximon und ein oder mehrere pharmazeutisch verträgliche nicht-ionischen Tenside in einer Menge von 10 bis 99 Gew.-% enthält.

US 2006/292213 beschreibt die Verwendung von Enoximon in der Behandlung von verschiedenen Krankheiten wie z.B. Asthma.

In der Literaturstelle Desmond J. Young, Gary A. Salzman "Status Asthmaticus in Adult Patients" werden verschiedene Möglichkeiten zur Behandlung von Status Asthmaticus beschrieben. Unter anderem werden auch Beta-2-Agonisten, Corticosteroide und Magnesiumsulfat genannt.

Da, wie oben beschrieben, bei Status asthmaticus die sonst üblichen therapeutischen Maßnahmen nur bedingt anwendbar sind, ist es Aufgabe der vorliegenden Erfindung, eine neue pharmazeutische Zusammensetzung bereitzustellen, die bei der Behandlung von Status asthmaticus, insbesondere bei der First-Line-Therapie und bei der Notfallbehandlung, eingesetzt werden kann und die die vorstehend genannten Nachteile und/oder komplizierte Eingriffe, wie eine künstliche Beatmung der Patienten, unnötig macht.

### Zusammenfassung der Erfindung

Es wurde erfindungsgemäß überraschenderweise erkannt, dass Status asthmaticus durch eine intravenöse Verabreichung von Enoximon behandelt werden kann. Dies gilt sowohl für Notfallpatienten mit einem Atemstillstand, als auch für Patienten, die noch atmen.

Die vorliegende Erfindung stellt daher eine pharmazeutische Zusammensetzung in Form einer injizierbaren Lösung zur Verwendung in der Behandlung von Status asthmaticus, d. h. von akutem schweren Asthma bronchiale, zur Verfügung, die den Wirkstoff Enoximon enthält. Die Lösung ist zur intravenösen Verabreichung ein oder mehrmals pro Tag vorgesehen, wobei so eine Menge von bis zu 300 mg Wirkstoff pro Tag und Patient verabreicht werden soll. Die pharmazeutische Zusammensetzung wird vorzugsweise in der Humanmedizin eingesetzt.

Mit anderen Worten betrifft die vorliegende Erfindung die Verwendung von Enoximon zur Herstellung eines Medikaments zur Behandlung von Status asthmaticus durch intravenöse Verabreichung.

Gemäß einer bevorzugten Ausführung der vorliegenden Erfindung wird der Wirkstoff Enoximon in einer Menge bzw. Dosis von 10 mg bis 300 mg pro Tag verabreicht bzw. ist die intravenöse Zusammensetzung zu einer Verabreichung von 10 mg bis 300 mg Enoximon pro Tag vorgesehen. Noch bevorzugter liegt die Untergrenze bei mindestens 20 mg, noch mehr bevorzugt bei mindestens 30 mg Enoximon pro Tag. Die Obergrenze liegt noch bevorzugter bei maximal 250 mg, noch bevorzugter bei 200 mg, noch bevorzugter bei maximal 180 mg, und noch mehr bevorzugt bei maximal 160 mg Enoximon pro Tag. Die angegebenen Mengen können einmal täglich als Einzeldosis oder in Form von mehreren Teildosen pro Tag verabreicht werden. Die angegebenen Werte beziehen sich auf die Menge an Wirkstoff pro Tag und Patient. Im Rahmen der vorliegenden Anmeldung wird vorzugsweise von einem Standardkörpergewicht eines Patienten von 70 kg ausgegangen. Sofern nachfolgend nicht explizit anders angegeben, beziehen sich auch dort die zu verabreichenden Mengen an Wirkstoff jeweils auf einen Patienten, vorzugsweise einen Patienten mit einem Standardkörpergewicht von 70 kg.

Bezieht man die zu verabreichende Wirkstoffmenge auf kg Körpergewicht des zu behandelnden Patienten und Tag, wird der Wirkstoff Enoximon gemäß einer weiteren bevorzugten Ausführungsform der vorliegenden Erfindung mit einer Dosis von 0,1 mg bis 5 mg Wirkstoff pro kg Körpergewicht und Tag verabreicht bzw. ist die intravenöse Zusammensetzung zu einer Verabreichung von 0,1 mg bis 5 mg Enoximon pro kg Körpergewicht und Tag vorgesehen. Noch bevorzugter liegt die Untergrenze bei 0,2 mg, noch bevorzugter bei 0,25 mg, noch bevorzugter bei 0,3 mg, und noch mehr bevorzugt bei 0,35 mg Enoximon pro kg Körpergewicht und Tag. Die Obergrenze liegt noch bevorzugter bei maximal 4,5 mg, noch bevorzugter bei 4 mg, noch bevorzugter bei 3,5 mg, noch bevorzugter bei 3 mg und noch mehr bevorzugt bei 2,5 mg Enoximon pro kg Körpergewicht und Tag. Die angegebenen Mengen können einmal täglich als Einzeldosis oder in Form von mehreren Teildosen pro Tag verabreicht werden.

Gemäß einer weiteren bevorzugten Ausführungsform der vorliegenden Erfindung beträgt eine zur Behandlung von Status asthmaticus zu verabreichende Einzeldosis von Enoximon, in Form einer Bolusinjektion, vorzugsweise einer Erstinjektion, beispielsweise im Rahmen einer Notfallversorgung bei einem Patienten mit Atemstillstand, oder einer Infusion, 25 mg, 30 mg, 50 mg, 100 mg, oder 200 mg pro Patient. Die Verabreichung von mehreren Einzeldosen mit gleicher oder unterschiedlicher Wirkstoffmenge pro Tag ist möglich.

Der Wirkstoff Enoximon kann in der Zusammensetzung in Wasser oder einer wässerigen Mischung gelöst sein. Gemäß einer Ausführungsform der vorliegenden Erfindung ist das Lösungsmittel Wasser. Gemäß einer weiteren Ausführungsform der vorliegenden Erfindung handelt es sich bei der wässerigen Mischung, um eine physiologisch annehmbare Salzlösung.

Der pharmazeutischen Zusammensetzung können übliche Zusatz- und Hilfsstoffe zugesetzt sein. Beispiele hierfür sind physiologisch verträgliche Lösungsvermittler und Stabilisatoren.

Als Lösungsvermittler können beispielsweise Propylenglykol, Polyole, wie Glycerin, und/oder Alkohole, wie beispielsweise Ethanol, eingesetzt werden. Durch den Zusatz von Lösungsvermittlern werden Lösungen erzielt, die trotz hoher Wirkstoffkonzentration in der Kälte stabil sind und nicht zu einer teilweisen Kristallisation des Wirkstoffs / der Wirkstoffe führen. Gemäß einer weiteren bevorzugten Ausführungsform wird Enoximon in einer Mischung aus Wasser und mindestens einem Lösungsvermittler gelöst.

Die Zusatz- und Hilfsstoffe können der verwendeten pharmazeutischen Zusammensetzung einzeln oder in einer beliebigen Kombination miteinander zugesetzt worden sein.

Gemäß einer besonders bevorzugten Ausführungsform der vorliegenden Erfindung liegt Enoximon gelöst in einer Mischung aus Wasser, Ethanol und Propylenglykol vor. Der pH-Wert der Lösung liegt vorzugsweise in einem Bereich von 12 bis 14, noch mehr bevorzugt in einem Bereich von 13 bis 14. Am meisten bevorzugt beträgt der pH-Wert der Enoximonenthaltenden Lösung 14. Zum Einstellen des pH-Werts können der Mischung geeignete Basen, beispielsweise Natriumhydroxid, zugesetzt werden. Gemäß einer weiteren Ausführungsform der vorliegenden Erfindung kann die wässerige Lösung aus Enoximon, Wasser, Ethanol, Propylenglykol und Natriumhydroxid bestehen.

Die Wirkstoff-Konzentration in der erfindungsgemäß eingesetzten Lösung bzw. Zusammensetzung beträgt vorzugsweise 1 bis 10 mg Enoximon pro ml Lösung. Noch mehr bevorzugt liegt die Untergrenze bei 2 mg, noch bevorzugter 4 mg Enoximon pro ml Lösung. Die Obergrenze liegt noch mehr bevorzugt bei 8 mg, noch bevorzugter bei 6 mg Enoximon pro ml Lösung. Gemäß einer besonders bevorzugten Ausführungsform beträgt die Wirkstoff-Konzentration 5 mg Enoximon pro ml Lösung.

Die Lösungen können als solches eingesetzt werden oder vor der Verabreichung weiter verdünnt werden.

Alle Bestandteile der pharmazeutischen Zusammensetzung sind in einem Reinheitsgrad, der für eine intravenöse Verabreichung geeignet ist. Auch die pharmazeutische Zusammensetzung als solche muss in einem entsprechenden Reinheitsgrad vorliegen. Entsprechende Inhaltsstoffe und Zusatzstoffe sind im Handel erhältlich. Ebenso sind dem Fachmann Maßnahmen zur Herstellung von Lösungen, die zur intravenösen Verabreichung geeignet sind, bekannt.

Die intravenöse Verabreichung der pharmazeutischen Zusammensetzung kann mittels Injektion, Infusion oder einer Kombination davon erfolgen. Gemäß einer Ausführungsform betrifft die vorliegende Erfindung also eine pharmazeutische, Enoximon-enthaltende Zusammensetzung zur Behandlung von Status asthmaticus durch intravenöse Injektion. Gemäß einer weiteren Ausführungsform betrifft die vorliegende Erfindung eine pharmazeutische, Enoximon-enthaltende Zusammensetzung zur Verwendung in der Behandlung von Status asthmaticus durch intravenöse Infusion. Eine noch weitere Ausführungsform der vorliegenden Erfindung betrifft eine pharmazeutische, Enoximon-enthaltende Zusammensetzung zur Verwendung in der Behandlung von Status asthmaticus durch eine Kombination aus intravenöser Injektion und intravenöser Infusion.

Unter Injektion wird hier das parenterale Einbringen der pharmazeutischen Zusammensetzung mittels einer Bolusinjektion verstanden. Für eine Injektion wird die Injektionslösung also beispielsweise in eine Spritze aufgezogen und in eine Vene appliziert. Die Injektionsgeschwindigkeit beträgt vorzugsweise 1 ml bis 10 ml Lösung pro Sekunde. Dies entspricht gemäß der vorstehend genannten Wirkstoff-Konzentrationen in der erfindungsgemäß eingesetzten Lösung bzw. Zusammensetzung einer Injektionsgeschwindigkeit von 1 mg bis 100 mg Enoximon pro Sekunde. Noch bevorzugter liegt die Injektionsgeschwindigkeit im Bereich von 2 ml bis 8 ml Lösung pro Sekunde, noch bevorzugter bei 4 ml bis 6 ml Lösung pro Sekunde. Die Injektionszeit liegt vorzugsweise in einem Bereich von 1 bis 10 Sekunden, noch bevorzugter in einem Bereich von 2 bis 8 Sekunden, und noch mehr bevorzugt in einem Bereich von 4 bis 6 Sekunden. Die Bolusinjektion kann ein oder mehrmals wiederholt werden. Die bei mehrmaligen Bolusinjektionen pro Bolusgabe verabreichte Menge an Enoximon kann gleichbleibend, ansteigend, abfallend oder eine beliebige Kombination davon sein. Der Abstand zwischen den einzelnen Injektionen liegt, je nach Zustand des Patienten, vorzugsweise im Bereich von 1 bis 30 Minuten, noch bevorzugter zwischen 2 und 20 Minuten. Ganz besonders bevorzugt zwischen 3 und 15 Minuten.

Im Rahmen einer Bolusinjektion können beispielsweise 100 mg Enoximon, das in einem Volumen von 20 ml gelöst ist, in einem Zeitraum von 4 bis 6 Sekunden injiziert werden. Eine derartige Darreichungs- und Dosierungsform kann beispielsweise zur Verwendung in der Behandlung von Status asthmaticus mit einhergehendem Atemstillstand vorgesehen sein.

Im Sinne der vorliegenden Patentanmeldung unterscheidet sich eine Infusion von einer Injektion dadurch, dass die pharmazeutische Zusammensetzung über einen längeren Zeitraum, vorzugsweise über einen Zeitraum von 1 bis 30 Minuten, verabreicht wird. Gemäß einer bevorzugten erfindungsgemäßen Ausführungsform handelt es sich bei der Infusion um eine kurzfristige Bolusinfusion über einen Zeitraum von 1 bis 15 Minuten. Noch bevorzugter beträgt der Zeitraum 2 bis 14 Minuten, noch bevorzugter 4 bis 12 Minuten, noch mehr bevorzugt 6 bis 10 Minuten.

Die Infusionsgeschwindigkeit liegt vorzugsweise im Bereich von 4 mg bis 16 mg Wirkstoff (= Enoximon) pro Minute, noch bevorzugter im Bereich von 8 mg bis 14 mg Wirkstoff pro Minute und am bevorzugtesten im Bereich von 8 mg bis 12 mg Wirkstoff pro Minute.

Im Rahmen einer kurzfristigen Bolusinfusion können beispielsweise 100 mg Enoximon über einen Zeitraum von 8 bis 12 Minuten, vorzugsweise 10 Minuten, injiziert werden. Eine derartige Darreichungs- und Dosierungsform kann beispielsweise zur Verwendung in der Behandlung von Status asthmaticus ohne Atemstillstand vorgesehen sein.

Die Infusion kann aber auch über einen längeren Zeitraum, beispielsweise über einen Zeitraum im Bereich von 16 bis 30 Minuten stattfinden. Die Infusionsgeschwindigkeit kann im Bereich von 1,5 bis 3 µg Wirkstoff pro kg Körpergewicht und Minute, bevorzugt bei 2,4 µg Wirkstoff pro Kg Körpergewicht und Minute, liegen.

Die Infusionsdauer wie auch die Infusionsgeschwindigkeit können an die zu verabreichende Wirkstoffmenge und die Wirkstoffkonzentration in der Infusionslösung angepasst werden.

Zur Steuerung der Dosierung kann eine handelsübliche, für einen Einsatz im medizinischen Bereich geeignete Spritzpumpe, beispielsweise ein Perfusor^{®}, eingesetzt werden.

Gemäß einer bevorzugten Ausführungsform der vorliegenden Erfindung wird der Wirkstoff bzw. die flüssige, den Wirkstoff enthaltende, pharmazeutische Zusammensetzung mittels einer einzigen intravenösen Bolusinjektion pro Tag und Patient verabreicht bzw. ist dazu vorgesehen, wobei die Enoximondosis vorzugsweise 25 mg, 30 mg, 50 mg oder 100 mg beträgt.

Die intravenöse Verabreichung ist besonders vorteilhaft, da in fortgeschrittenen Fällen einer Bronchokonstriktion eine Nebulisierung von Wirkstoffen fehlschlägt, da die inhalierten Arzneimittel bzw. Wirkstoffe die Alveoli nicht erreichen können.

Die erfindungsgemäß eingesetzten Injektionspräparate können für die therapeutische Anwendung nach üblichen Verfahren sterilisiert oder keimfrei in geeignete Behälter, wie beispielsweise Septumflaschen oder Ampullen, abgefüllt werden, die beispielsweise 100 mg Enoximon in 20 ml Gesamtvolumen enthalten. Die sonstigen Bestandteile der Zusammensetzung, neben dem Wirkstoff Enoximon, sind vorzugsweise Ethanol, noch bevorzugter wasserfreies Ethanol, Natriumhydroxid, Propylenglykol und Wasser. Der pH-Wert der Zusammensetzung liegt vorzugsweise im Bereich von 12 bis 14, und beträgt noch bevorzugter 14.

Die Injektions- oder Infusionspräparate können als gebrauchsfertige Lösungen vorliegen. Alternativ können die Behälter ein Konzentrat des Injektions- oder Infusionspräparats enthalten, das dann zur Herstellung einer normalen Injektions- oder Infusionslösung verdünnt werden kann. Die Verdünnungsfaktor liegt vorzugsweise im Bereich von 1:2, also ein Teil Konzentrat und ein Teil Verdünnungsmittel, bis zu 1:10, noch mehr bevorzugt 1:8. Das Verdünnungsmittel ist vorzugsweise Wasser für Injektionszwecke oder eine physiologisch annehmbare Salzlösung.

Gemäß einer weiteren erfindungsgemäßen Ausführungsform kann Enoximon auch in Kombination mit weiteren Wirkstoffen, die zur Verwendung in der von Asthma bronchiale eingesetzt werden, eingesetzt werden. Der weitere Wirkstoff kann vorzugsweise ausgewählt sein aus der Gruppe, bestehend aus Beta-2-Sympathomimetika, Katecholaminen, Aminophyllinen, Corticoide, Parasympatholytika, Magnesiumsulfaten, Ketamin, S-Ketamin und Kombinationen davon. Ganz besonders bevorzugt wird Enoximon in einer Kombination mit dem Glucocorticoid Prednisolon und Magnesiumsulfat eingesetzt.

Die vorliegende Erfindung betrifft somit ferner einen Kit enthaltend eine erfindungsgemäße pharmazeutische Zusammensetzung und einen Wirkstoff ausgewählt aus der Gruppe, bestehend aus Beta-2-Sympathomimetika, Katecholaminen, Aminophyllinen, Corticoiden, Parasympatholytika, Magnesiumsulfaten, Ketamin, S-Ketamin und Kombinationen davon zur Verwendung in der Behandlung von Status asthmaticus.

Es hat sich überraschenderweise gezeigt, dass eine intravenöse Verabreichung von Enoximon eine sehr effektive Behandlungsweise von Status asthmaticus darstellt. Insbesondere ist die intravenöse Verabreichung dazu geeignet, eine künstliche Beatmung und daher auch die mit dieser Behandlung auftretenden Komplikationen zu vermeiden. Es zeigte sich auch, dass ein Bronchospasmus innerhalb von 5 bis 15 Minuten gelöst werden konnte. Weiterhin zeigte es sich, dass es bei Patienten mit akutem schwerem Asthma bronchiale nicht mehr nötig war, diese auf die Intensivstation zu verlegen.

### Beispiele

Im folgenden wird die Erfindung anhand von sieben Patientenberichten näher erläutert, ohne diese jedoch darauf einzuschränken.

Allen Patienten wurde eine im Handel unter dem Namen Perfan^{®} i.v. erhältliche Enoximon-Lösung verabreicht, die formulierungstechnisch nicht verändert wurde. Die eingesetzte Lösung enthielt Enoximon in einer Konzentration von 5 mg/ml, Natriumhydroxid, wasserfreies Ethanol, Propylenglykol und Wasser für Injektionszwecke.

Die Blutgasanalyse wurde nach dem üblichen Standardverfahren durchgeführt.

Die folgenden sieben Patientenstudien betreffen Patienten mit Status asthmaticus, die auf die Intensivstation zur künstlichen Beatmung eingeliefert wurden.

### Erster Patientenbericht

Die erste Patientin war eine 34 Jahre alte Frau mit einer bekannten Historie mit schwerem Asthma und atopischem Ekzem. Ihre Schwester war bereits an einem Asthmaanfall gestorben. Die Patientin wurde in der Notfallaufnahme mit einem Status asthmaticus eingeliefert und hatte bei ihrem eintreffen einen Atemstillstand. Die Kapillarblutgas-Messung in der Notfallaufnahme ergab folgende Werte: pH 7,07; pCO₂ 92 mmHg; Basenüberschuss - 4,1; act bicarb 26,1; pO₂ 36 mmHg; satO₂ 46%. Sie wurde sofort intubiert und auf die Intensivstation verlegt. Sie erhielt Salbutamol/Ipratropiumbromid Zerstäuber und Aminophyllin, jedoch ohne dass diese Medikamente einen wesentlichen Einfluss hatten. Die künstliche Beatmung war nahezu unmöglich, da nur ein Minutenvolumen von 2,5 Litern möglich war. Das erste arterielle Blutgas-Messung auf der Intensivstation lieferte folgende Werte: pH 7,08; pCO₂ 80 mmHg; Basenüberschuss -6,5; act bicarb 23,4; pO₂ 143 mmHg; satO₂ 98%. In Anbetracht dieser sehr ernsthaften Situation erhielt die Patientin eine intravenöse Bolusinjektion von 100 mg Enoximon, das innerhalb von 5 Sekunden verabreicht wurde. Der Wirkstoff war in einem Gesamtvolumen von 20 ml gelöst. Innerhalb von 5 Minuten darauf war das Minutenvolumen 14 Liter. Eine Stunde später war das Blutgas: pH 7,24; pCO₂ 55 mmHg; Basenüberschuss -4,2; pO₂ 167 mmHg; satO₂ 99%. Danach wurde die Behandlung mit einer Spritzpumpe (Perfusor^{®}) und zwar mit 200 mg Enoximon pro Tag fortgesetzt. Nach fünf Tagen konnte sie von der künstlichen Beatmung abgesetzt werden. Nach einem insgesamt 44-tägigen Krankenhausaufenthalt konnte sie nach Hause entlassen werden.

Erstaunlicherweise konnte beobachtet werden, dass mit der Gabe von Enoximon die Bronchokonstriktion aufgehoben wurde. Die Behandlung der Patientin mit akutem Asthma mit Enoximon erfolgte durch eine sofortige Gabe von Enoximon.

### Zweiter Patientenbericht

Der zweite Patient war ein 59 Jahre alter Mann mit Asthma, der in der Lungenabteilung mit ansteigender schwerer Atemlosigkeit eingeliefert wurde. Er erhielt Augmentin zur Behandlung einer Atemwegsinfektion, Salbutamol und Ipratropiumbromid und Prednisolon. Nach ein paar Stunden wurde er auf die Intensivstation verlegt. Er erhielt dort Enoximon als intravenöse Inkrementgabe, d.h. mehrere kleine Bolusinjektionen mit 10 und 20 mg Wirkstoff, mit insgesamt 50 mg Wirkstoff in 15 Minuten. Nach fünf Minuten fühlte sich der Patient besser und war in der Lage, wieder volle Sätze zu sprechen. Der Patient wurde nicht intubiert und zwei Tage später wurde er zurück in die Lungenabteilung verlegt. Nach einer Verweilzeit von zehn Tagen verließ er das Krankenhaus.

### Dritter Patientenbericht

Der dritte Patient war ein 74 Jahre alter Mann mit Asthma, Goldklasse II. Er wurde in die Lungenabteilung mit ansteigender Kurzatmigkeit eingeliefert. Zwei Wochen zuvor hatte er Doxycylin und Prednisolon zur Behandlung einer Atemwegsinfektion erhalten. Zehn Stunden nach Aufnahme im Krakenhaus wurde er auf die Intensivstation zum Absaugen eingeliefert. Er erhielt insgesamt 30 mg Enoximon, das intravenös mittels zweier Boli von je 15 mg Wirkstoff verabreicht wurde. Der Patient erholte sich sehr schnell und zwei Stunden danach wurde er auf die Lungenstation zurückverlegt. Nach insgesamt neun Tagen Behandlung verließ er das Krankenhaus.

### Vierter Patientenbericht

Der vierte Patient war ein 67 Jahre alter Mann mit Asthma. Er wurde in die Lungenabteilung mit ansteigender Kurzatmigkeit eingeliefert, die während der letzten fünf Tage schon angedauert hatte. Sein Zustand verschlechterte sich rapide und er wurde auf die Intensivstation verlegt. Dort erhielt er insgesamt 100 mg Enoximon, das in drei Bolusgaben von 25 mg, 25 mg und 50 mg Wirkstoff intravenös verabreicht wurde. Der Patient wurde intubiert und beatmet. Nach 15 Minuten erhielt er einen Extrabolus von 100 mg Enoximon gefolgt von einer intravenösen Infusion (Dauerinfusion, d.h. 10 mg / Stunde, über 24 Stunden). Danach konnte er von der künstlichen Beatmung weggenommen werden und wurde am nächsten Tag extubiert. Die Krankenhausbehandlung dauerte 17 Tage.

### Fünfter Patientenbericht

Der fünfte Patient war ein 34 Jahre alter Mann. Er hatte seine Asthmabehandlung abgesetzt und bekam daher eine schwere Kurzatmigkeit. Er wurde auf die Notfallstation mit Status asthmaticus eingeliefert. Er erhielt 100 mg Enoximon, das intravenös mittels einmaliger Bolusinjektion verabreicht wurde, Prednisolon und Magnesiumsulfat. Sofort danach wurde er auf die Intensivstation verlegt. Er benötigte keine künstliche Beatmung und am nächsten Tag konnte er auf die Lungenstation verlegt werden. Der Krankenhausaufenthalt dauerte acht Tage.

### Sechster Patientenbericht

Der sechste Patient war ein 16 Jahre alter Mann, der von seinen Eltern in die Notfallstation gebracht wurde. Er hatte eine starke Kurzatmigkeit und es bestand keine Möglichkeit der Nebulisation. Er erhielt eine intravenöse Bolusinjektion von 100 mg Enoximon, das innerhalb von 5 Sekunden verabreicht wurde. Der Wirkstoff war in einem Gesamtvolumen von 20 ml gelöst. Dies wurde nach fünf Minuten wiederholt. Zehn Minuten nach dem letzten Bolus war es wieder möglich, dass er ganze Sätze sprach. Der Patient hatte Blutgaswerte bei seiner Einlieferung von pH 6,98; pCO₂ 113 mmHg; Basenüberschuss -5,0; act-HCO₃ 26,6 mmol/l; pO₂ 63 mmHg; sO₂ 82%. 91 Minuten später waren seine Blutgaswerte in der Intensivstation normal und zwar pH 7,35; pCO₂ 40 mmHg; Basenüberschuss -3,5; act-HCO₃ 22,1 mmol/l; pO₂ 125 mmHg; sO₂ 98%.

### Siebter Patientenbericht

Der siebte Patient war ein 52 Jahre alter Mann mit Asthma, der in die Lungenabteilung mit ansteigender Kurzatmigkeit eingeliefert wurde. Es war bekannt, dass dieser Patient zu Hause nicht mehr auf die Therapie mit Spiriva^{®} und Atimos^{®} ansprach. Er wurde mit Prednisolon iv und Salbutamol/Ipratropiumbromid Zerstäuber behandelt. Einige Stunden nach seinem Zugang wurde er auf die Intensivstation mit sich noch verschlechternder Kurzatmigkeit und einer Atemfrequenz von 50/min eingeliefert. Kurz nach seinem Zugang stoppte seine Atmung. Er erhielt eine intravenöse Bolusinjektion von 100 mg Enoximon, das innerhalb von 5 Sekunden verabreicht wurde. Der Wirkstoff war in einem Gesamtvolumen von 20 ml gelöst. Der Patient wurde außerdem für einige Minuten künstliche beatmet. Danach gewann er das Bewusstsein wieder und es bestand keine Notwendigkeit zur Intubation.

Von den oben beschriebenen sieben Patienten können fünf in die Gruppe von fatalem Asthma eingeordnet werden, da ihre Atmung aussetzte und zwei Patienten können einklassifiziert werden in bedrohliches Asthma gemäß den British *Guidelines* on *the Management of Asthma, revised June 2009.*

Es zeigt sich, dass sich die Behandlung von Status asthmaticus bei dekompensiertem Bronchialasthma eine Herausforderung für den behandelnden Arzt darstellt. Die klassischen Behandlungsmethoden, wie eine Nebulisierung eines Beta-2-mimetischen Wirkstoffes, Nebulisierung mit anticholinergenen Wirkstoffen, Behandlung mit intravenösen Katecholaminen, Aminophyllin, Corticosteroiden, Magnesiumsulfaten, Ketaminen oder S-Ketaminen sind begrenzt. So kann in fortgeschrittenen Fällen einer Bronchokonstriktion eine Nebulisierung fehlschlagen, da die inhalierten Drogen bzw. Wirkstoffe niemals die Alveoli erreichen können. Die Verwendung von Aminophyllin und Beta-2-mimetischen Wirkstoffen ist ebenfalls aufgrund von ansteigender Kurzatmigkeit limitiert. Auch eine künstliche Beatmung sollte wenn möglich, aus den vorstehend genannten Gründen und Problemen vermieden werden.

Die erfindungsgemäße Gabe von Enoximon mittels einer injizierbaren Lösung hat daher überraschenderweise eine neue Lösung für die Behandlung von Status asthmaticus geschaffen. Wie sich beispielsweise bei dem ersten Patienten gezeigt hat, wurden 100 mg Enoximon sehr schnell verabreicht. Zur Überraschung reagierte der Patient innerhalb der ersten fünf Minuten und konnte normal beatmet werden. Die erhaltenen Ergebnisse zeigen, dass niemand der Patienten einen hämodynamischen Vorfall wie Tachykardie oder schwere Hypertension zeigte.

Eine intravenöse Gabe von Enoximon stellt daher erfindungsgemäß eine sehr gute Erstbehandlung von Status asthmaticus dar und kann auch die Notwendigkeit von künstlicher Beatmung verhindern, so dass auch weniger Komplikationen bei der Behandlung derartiger Patienten auftreten können. Bei einer erfolgreichen Behandlung mit Enoximon kann es daher auch überflüssig werden, die Patienten auf die Intensivstation zu verlagern.

## Patentansprüche

1. Pharmazeutische Zusammensetzung, enthaltend Enoximon, zur Verwendung in der Behandlung von Status asthmaticus durch intravenöse Verabreichung ein- oder mehrmals pro Tag in einer Menge von bis zu maximal 300 mg Enoximon pro Tag, vorzugsweise in einer Menge von 200 mg Enoximon pro Tag.

2. Pharmazeutische Zusammensetzung gemäß der Verwendung von Anspruch 1 als wässerige Lösung oder als wässeriges Lösungsmittelgemisch, vorzugsweise unter Zusatz von physiologisch verträglichen Lösungsvermittlern und/oder Stabilisatoren.

3. Pharmazeutische Zusammensetzung gemäß der Verwendung von Anspruch 2, wobei die wässerige Lösung Enoximon, Wasser, Ethanol, Propylenglykol und Natriumhydroxid enthält.

4. Pharmazeutische Zusammensetzung gemäß der Verwendung von einem der vorstehenden Ansprüche, zur Verabreichung durch intravenöse Injektion.

5. Pharmazeutische Zusammensetzung gemäß der Verwendung von Anspruch 4, wobei Enoximon mit einer Dosis von 25 mg, 30 mg, 50 mg oder 100 mg verabreicht wird.

6. Pharmazeutische Zusammensetzung gemäß der Verwendung von Anspruch 4 oder 5, wobei Enoximon mit einer Menge von 100 mg in einem Gesamtvolumen von 20 ml verabreicht wird und die Injektionsgeschwindigkeit 5 ml Lösung pro Sekunde beträgt.

7. Pharmazeutische Zusammensetzung gemäß der Verwendung von Anspruch 4, wobei Enoximon mit einer Menge von 100 mg in einem Gesamtvolumen von 40 ml verabreicht wird und die Infusionsgeschwindigkeit 10 mg Enoximon pro Minute beträgt.

8. Kit, enthaltend eine pharmazeutische Zusammensetzung gemäß einem der vorhergehenden Ansprüche 1 bis 7 und einen Wirkstoff ausgewählt aus der Gruppe, bestehend aus Beta-2-Sympathomimetika, Katecholaminen, Aminophyllinen, Corticoiden, Parasympatholytika, Magnesiumsulfaten, Ketamin, S-Ketamin und Kombinationen davon zur Verwendung in der Behandlung von Status asthmaticus.

## Claims

1. A pharmaceutical composition comprising enoximone for use in the treatment of status asthmaticus by intravenous administration one or several times per day in an amount of up to a maximum of 300 mg enoximone per day, preferably in an amount of 200 mg enoximone per day.

2. The pharmaceutical composition according to the use of claim 1 as an aqueous solution or as an aqueous solvent mixture, preferably with the addition of physiologically acceptable solubilizers and / or stabilizers.

3. The pharmaceutical composition according to the use of claim 2, wherein the aqueous solution comprises enoximone, water, ethanol, propylene glycol and sodium hydroxide.

4. The pharmaceutical composition according to the use of any one of the preceding claims for administration by intravenous injection.

5. The pharmaceutical composition according to the use of claim 4, wherein enoximone is administered with a dosage of 25 mg, 30 mg, 50 mg or 100 mg.

6. The pharmaceutical composition according to the use of claim 4 or 5, wherein enoximone with an amount of 100 mg in a total volume of 20 ml is administered, and the injection speed is 5 ml solution per second.

7. The pharmaceutical composition according to the use of claim 4, wherein enoximone with an amount of 100 mg in a total volume of 40 ml is administered, and the infusion speed is 10 mg enoximone per minute.

8. A kit comprising a pharmaceutical composition according to any one of the preceding claims 1 to 7 and an active ingredient selected from the group consisting of beta-2 sympathomimetic drugs, catecholamines, aminophyllines, corticoids, parasympatholytics, magnesium sulfates, ketamine, S-ketamine, and combinations thereof for use in the treatment of status asthmaticus.

## Revendications

1. Composition pharmaceutique contenant de l'énoximone, destinée à l'utilisation dans le traitement de l'état de mal asthmatique en administrant par voie intraveneuse, une ou plusieurs fois par jour, une quantité pouvant atteindre jusqu'à 300 mg d'énoximone par jour, de préférence une quantité de 200 mg d'énoximone par jour.

2. Composition pharmaceutique selon l'utilisation de la revendication 1, sous forme d'une solution aqueuse ou d'un mélange de solvants aqueux, réalisée de préférence en y ajoutant des agents de solubilisation et/ou des agents stabilisateurs physiologiquement compatibles.

3. Composition pharmaceutique selon l'utilisation de la revendication 2, ladite solution aqueuse contenant de l'énoximone, de l'eau, de l'éthanol, du propylène glycol et de l'hydroxyde de sodium.

4. Composition pharmaceutique selon l'utilisation de l'une des revendications précédentes, destinée à l'administration par injection intraveneuse.

5. Composition pharmaceutique selon l'utilisation de la revendication 4, ladite énoximone étant administrée en une dose de 25 mg, 30 mg, 50 mg ou de 100 mg.

6. Composition pharmaceutique selon l'utilisation des revendications 4 ou 5, ladite énoximone étant administrée en une quantité de 100 mg comprise dans un volume total de 20 ml, et la vitesse d'injection étant de 5 ml de solution par seconde.

7. Composition pharmaceutique selon l'utilisation de la revendication 4, ladite énoximone étant administrée en une quantité de 100 mg comprise dans un volume total de 40 ml, et la vitesse de perfusion étant de 10 mg d'énoximone par minute.

8. Kit, contenant une composition pharmaceutique selon l'une des revendications précédentes 1 à 7 et un principe actif choisi dans le groupe constitué de bêta-2-sympathomimétiques, de catécholamines, d'aminophyllines, de corticoïdes, de parasympatholytiques, de sulfates de magnésium, de la kétamine, de la S-kétamine et de leurs combinaisons, destiné à l'utilisation dans le traitement de l'état de mal asthmatique.
